# EUROPEAN PATENT APPLICATION

(11) **EP 4 772 583 A1**
(43) Date of publication of application: **08.07.2026**
(21) Application number: 24859479.8
(22) Date of filing: 15.08.2024
(51) Int. Cl.: C09B 67/02, A61K 8/44, A61K 8/96, A61Q 1/02, A61Q 1/04, A61Q 1/10, A61Q 3/02, A61Q 5/10, B41M 5/00, C09B 61/00, C09D 7/41, C09D 11/30, C09D 201/00

(54) **WATER-INSOLUBLE PIGMENT COMPOSITION**

(30) Priority: 31.08.2023 JP 2023140961; 28.03.2024 JP 2024053339
(71) Applicant: DIC Corporation, Tokyo 174-8520 (JP)
(72) Inventor: SEKIKAWA, Hiroshi, Kamisu-shi, Ibaraki 314-0193 (JP); TANAKA, Masaaki, Kamisu-shi, Ibaraki 314-0193 (JP); YASUI, Kengo, Kamisu-shi, Ibaraki 314-0193 (JP)
(74) Representative: TBK
(86) International application number: PCT/JP2024/029039
(87) International publication number: WO 2025/047443

(57) **Abstract**

The present invention is directed to providing a water-insoluble pigment composition obtained by complexing a natural pigment and a dehydration condensation compound of a fatty acid and an amino acid, and a food, a cosmetic, a lipstick cosmetic, an eye cosmetic, a nail cosmetic, a base makeup cosmetic, a hair cosmetic, a pharmaceutical, a coating material for agrochemicals, a printing marker, a stationery, a writing instrument, a printing ink, an inkjet ink, a metal ink, a paint, a plastic pigment, a color toner, a fluorescent labeling agent, a fluorescent probe, or a chemical sensor containing the water-insoluble pigment composition. **It** has been found that a water-insoluble pigment composition is obtained by complexing a natural pigment and a dehydration condensation compound of a fatty acid and an amino acid, leading to completion of the present invention.

## Description

### TECHNICAL FIELD

The present invention relates to a water-insoluble pigment composition.

### BACKGROUND ART

There are a wide variety of red pigments, yellow pigments, and blue pigments as natural pigments, but in recent years, synthetic pigments have been questioned due to problems such as carcinogenicity, and there is a growing expectation for natural pigments that are considered to be safer.

In addition, business activities strongly conscious of sustainability have been attracting attention, and environmentally friendly coloring materials have been required. Under such circumstances, many natural pigments are easily dissolved in water, and thus, when used in cosmetics or food pigments, there are problems of elution into water and discoloration accompanying the elution. Further, in a case where natural pigments are used for flexographic printing, problems such as migration and plate staining occur. Accordingly, natural pigments are used only in a very limited application at present.

The inventors have investigated documents describing solutions to the problems of elution of natural pigments into water and discoloration, that is, improvement of water solubility, and have found that there is a description relating to a method for extracting natural pigments, and there is a description relating to orally administered cosmetics, dairy products, and pet foods using the extracts (Cited Document 1). Further, there is a description that, for the purpose of stabilizing the hue, a carotenoid-based pigment, sodium tartrate, alum, and sodium carbonate are dry-blended, and the mixed powder is dissolved in water to obtain an aqueous solution (Cited Document 2). In addition, there is a description of a powder composition containing a carotenoid, an oily component, and a sugar (Cited Document 3).

However, these documents do not improve the water solubility of natural pigments. It is an aspired problem to improve the water solubility of natural pigments.

### CITATION LIST

### PATENT DOCUMENT

Patent Document 1: JP 2009-508505 T
Patent Document 2: JP S59-050264 B
Patent Document 3: JP 2009-185023 A

### SUMMARY OF INVENTION

### TECHNICAL PROBLEM

The present invention is directed to providing a water-insoluble pigment composition, and a food, a cosmetic, a coating material for a pharmaceutical or an agrochemical, or a printing marker, a stationery, a writing instrument, a printing ink, an inkjet ink, a metal ink, a paint, a plastic pigment, a color toner, a fluorescent labeling agent, a fluorescent probe, or a chemical sensor containing the water-insoluble pigment composition.

### SOLUTION TO PROBLEM

The present inventors have conducted intensive studies to solve the above problem, and as a result, have found that a water-insoluble pigment composition is obtained by complexing a natural pigment, and a dehydration condensation compound of a fatty acid and an amino acid, thereby completing the present invention.

That is, the present invention encompasses the following aspects.
[1] A water-insoluble pigment composition obtained by complexing a natural pigment, and a dehydration condensation compound of a fatty acid and an amino acid.
[2] The water-insoluble pigment composition according to 1, wherein a composition of the natural pigment and the dehydration condensation compound of the fatty acid and the amino acid satisfies, in terms of mass ratio, natural pigment: dehydration condensation compound of fatty acid and amino acid = 0.1 : 99.9 to 90 : 10.
[3] The water-insoluble pigment composition according to 1 or 2, wherein the dehydration condensation compound of the fatty acid and the amino acid is one or more selected from lauroyl lysine and N-capryloyl lysine.
[4] The water-insoluble pigment composition according to 1 or 2, wherein the natural pigment is at least one or more selected from a carotenoid-based pigment, a porphyrin-based pigment, a flavonoid-based pigment, and a chromoprotein.
[5] The water-insoluble pigment composition according to 1 or 2, wherein the natural pigment is at least one or more selected from annatto pigment, chlorophyll pigment, safflower yellow pigment, and phycocyanin pigment.
[6] A food, a cosmetic, a lipstick cosmetic, an eye cosmetic, a nail cosmetic, a base makeup cosmetic, a hair cosmetic, a pharmaceutical, a coating material for agricultural chemicals, a printing marker, a stationery, a writing instrument, a printing ink, an inkjet ink, a metal ink, a paint, a plastic pigment, a color toner, a fluorescent labeling agent, a fluorescent probe, or a chemical sensor, containing the water-insoluble pigment composition according to 1 to 5.

### ADVANTAGEOUS EFFECTS OF INVENTION

According to the present invention, a water-insoluble pigment composition can be provided. The water-insoluble pigment composition of the present invention has high water resistance, and thus, it is less likely to be eluted into water and to lose its color due to the elution when used for a cosmetic or a food. In addition, in a case where the water-insoluble pigment composition is used for flexographic printing, migration and plate staining are reduced.

### DESCRIPTION OF EMBODIMENTS

Hereinafter, the water-insoluble pigment composition of the present invention will be described in detail, but the description of constituent elements described below is an example as one embodiment of the present invention, and the present invention is not limited to the contents thereof.

### Natural Pigment

The natural pigment to be used in the present invention may be any pigment derived from nature, such as red cabbage pigment, red radish pigment, annatto pigment, squid ink pigment, turmeric pigment, cacao pigment, carotene pigment, carotenoid pigment, gardenia red pigment, gardenia blue pigment, gardenia yellow pigment, chlorophyll pigment, kaoliang pigment, cochineal pigment, saffron pigment, perilla pigment, cythane pigment, spirulina pigment, phycocyanin pigment, onion pigment, tamarind pigment, butterfly pea pigment, chili pepper pigment, tomato pigment, hibiscus pigment, beet red pigment, grape berry skin pigment, Haematococcus pigment, red yeast rice pigment, safflower red pigment, safflower yellow pigment, berry pigment, marigold pigment, purple sweet potato pigment, purple corn pigment, purple yam pigment, caramel pigment, and plant charcoal powder pigment. Those obtained by biosynthesis, enzymatic synthesis, or chemical synthesis of these pigments can also be used in the same manner.

Among them, examples of the pigment that can be suitably used in the present invention include a carotenoid-based pigment, a porphyrin-based pigment, a flavonoid-based pigment, and chromoprotein.

As the carotenoid-based pigment, a red pigment extracted from carrot, tomato, or red pepper, a yellow pigment extracted from citrus fruit, gardenia, annatto, or marigold, and the like are known, and a large amount of carotenoid-based pigments are also contained in green leaf vegetables.

A carotenoid is a substance belonging to tetraterpenes among terpene compounds, terpenes are important compounds for plants, and 20000 or more compounds thereof are known. As the carotenoid-based pigment, hydrocarbon-based pigments such as β-carotene and lycopene, xanthophyll-based pigments such as astaxanthin, capsanthin, and lutein, bixin, norbixin, crocin, and the like are known.

The porphyrin-based pigment has four pyrrole rings, and a metal complex thereof is contained in chlorophyll that plays a role of light absorption and photoelectron transfer in photosynthesis, heme of hemoglobin that transports oxygen in blood, and the like, and is a compound that plays an important role in a living body. The porphyrin metal complex is used in various fields as an optoelectronic functional material, a metal complex catalyst, and a molecular conductive material, and is known to exhibit a wide variety of functionalities by changing a peripheral substituent, a central metal, and an axial ligand of porphyrin.

Flavonoid is a large class of polyphenols. Flavonoid is a generic name of components having a certain chemical structure, is contained in leaves, stems, trunks, and the like of plants, is a substance produced by plants to protect themselves from ultraviolet rays, pests, and the like, and is a source of pigments and bitter components. Flavonoid is classified into flavonols, flavones, catechins, flavanones, anthocyanins, isoflavones, and the like, depending on the difference in structure.

Examples of the flavonoid-based pigment include flavonol, anthocyanin pigments exhibiting various color tones, and pigments such as chalcone and aurone that develop a deep yellow color.

### Chromoprotein

Chromoprotein is a generic term for proteins that are complexed with a pigment in a natural state. Chromoprotein is present in cells and body fluids of animals and plants, and develops various color tones and physiological functions by prosthetic groups including pigments. Hemoprotein is a conjugate of an iron-porphyrin complex salt and a protein, and the binding ratio of the protein and the heme is 1 : 1, 1 : 2, 1 : 4, or the like. Hemoprotein is widely found in nature and examples thereof include hemoglobin, myoglobin, cytochrome, catalase, and peroxidase.

A metal complex compound is a conjugate of a metal complex ion and a protein, and examples thereof include a copper protein such as hemocyanin and an iron protein such as ferritin. Ferritin is present in the spleen, small intestine mucosa, liver, and the like, and is considered to be involved in the storage of iron in vivo and the absorption of iron during digestion.

A phycochromoprotein is a conjugate of a pyrrole derivative and a protein, and examples thereof include phycoerythrin exhibiting a red color of a red alga plant, and phycocyanin exhibiting a blue color of a blue alga plant. These are contained in chloroplasts together with chlorophyll and a carotenoid, and are considered to be auxiliary pigments for photosynthesis.

A flavoprotein has flavin mononucleotide or flavin adenine dinucleotide as a prosthetic group. All of them have a function as an oxidoreductase and are also called flavoenzymes. Examples thereof include amino acid oxidase and xanthine oxidase.

A carotenoid protein is a conjugate of a carotenoid and a protein. There is a conjugate of vitamin A and a protein, and rhodopsin is one of them.

### Phycocyanin

Phycocyanin to be used in the present invention is a chromoprotein, which has phycocyanobilin as a chromophore. Phycocyanin has a structure in which phycocyanobilin and a protein are bonded to each other.

Examples of the phycocyanin according to the present invention include phycocyanins derived from algae such as phycocyanins derived from blue algae, phycocyanins derived from red algae, and phycocyanins derived from cryptoalgae, and among these, phycocyanins derived from blue algae are preferable because they can be collected in large amounts.

Examples of the blue algae include blue algae of the genus Spirulina, the genus Arthrospira, the genus Aphanizomenon, the genus Fisherella, the genus Anabaena, the genus Nostoc, the genus Synechocystis, the genus Synechococcus, the genus Tolypothrix, the genus Aphanothece, and the genus Mastigoclaus, the genus Pleurocapsa. Among them, blue algae of the genus Spirulina and the genus Arthrospira, which are produced on an industrial scale and whose safety has been confirmed, are preferable, and blue algae of the genus Spirulina are more preferable.

Raw blue algae or dried blue algae may be used as a raw material for preparing phycocyanin. The dried product of blue algae may be obtained by drying raw blue algae according to a common method, or a commercially available dried product may be used.

Phycocyanin can be obtained by, for example, suspending blue algae in water or a buffer solution such as a phosphate buffer solution or a citrate buffer solution and extracting phycocyanin in the blue algae.

A method for extracting phycocyanin is not particularly limited, and a generally known method can be used.

A preferred embodiment of the extraction method is, for example, the extraction method described in JP 2006-230272 A. Specific examples thereof include an extraction method described in the following extraction method (i). With such an extraction method (i), it is possible to obtain phycocyanin having high purity and a bright color tone.

### Extraction Method (i)

The extraction method (i) includes:
a first step of obtaining an extraction liquid in which phycocyanin in blue algae is extracted into an aqueous suspension;
a second step of reacting a calcium salt with a phosphate in the extraction liquid to generate calcium phosphate and adsorbing impurities of phycocyanin to the calcium phosphate to obtain an adsorbed substance; and a third step of removing a residue of the blue algae and the adsorbed substance from the extraction liquid.

Furthermore, the extraction method (i) is more preferably the following extraction method (ii).

### Extraction Method (ii)

The extraction method (ii) includes:
a first step of obtaining an extraction liquid in which phycocyanin in blue algae is extracted into an aqueous suspension;
a second step of reacting a calcium salt with a phosphate in the extraction liquid to generate calcium phosphate and adsorbing impurities of phycocyanin to the calcium phosphate to obtain an adsorbed substance;
a third step of removing a residue of the blue algae and the adsorbed substance from the extraction liquid; and
a step of causing the extraction liquid to contain a chelating agent before the third step.

The phycocyanin to be used in the present invention is a commercial product LINABLUE G1 (available from DIC LIFETECH CORPORATION, trehalose 55%, spirulina extract 40%, trisodium citrate 5%) mixed with a stabilizer. As described in JP H11-299450 A, as for these components, trehalose is used to increase thermal stability, and citric acid is used as a pH adjuster. Note that a phycocyanin pigment is contained as a main component in a spirulina extract.

In the present invention, the natural pigment may be used alone or in combination of several kinds thereof. To adjust a color to a desired hue depending on the application, natural pigments may be mixed in an advance and complexed with the dehydration condensation compound of a fatty acid and an amino acid, or respective single natural pigments may be complexed with the dehydration condensation compound of a fatty acid and an amino acid, and then the complexed water-insoluble pigment compositions may be mixed.

### Dehydration Condensation Compound of Fatty Acid and Amino Acid

A compound generated by dehydration condensation of a carboxyl group of a fatty acid and an amino group of an amino acid is generally used for the purpose of applications such as a surfactant. An amino acid and a fatty acid are constituents of a living body, and are substances that have excellent biodegradability and have few adverse effects on the living body. Thus, the dehydration condensation compound of a fatty acid and an amino acid generated from these substances as raw materials can be expected to have safety and biodegradability. Examples of the dehydration condensation compound of a fatty acid and an amino acid to be used in the present invention include lauroyl lysine and N-capryloyl lysine.

Lauroyl lysine is an amide compound generated by dehydration condensation of a carboxyl group of lauric acid and an amino group of lysine, and is a component frequently used in makeup cosmetics including a foundation and a lipstick. It is a highly safe compound which is used for texture improvement and surface treatment purposes to provide high transparency and quality. In addition, lauroyl lysine is characterized by being hardly dissolved in a solvent or water. However, it is easily dissolved in an alkaline aqueous solution.

N-capryloyllysine is an amide compound generated by dehydration condensation of a carboxyl group of caprylic acid and an amino group of glycine. Similarly to lauroyl lysine, it is a highly safe compound often used in cosmetic applications.

In the present invention, any dehydration condensation compound of a fatty acid and an amino acid may be used, but the average particle size thereof is preferably from 0.1 µm to 100 µm, more preferably from 1 µm to 50 µm, and still more preferably from 5 µm to 30 µm. The particle size is preferably spherical or plate-like.

### Water-Insoluble Pigment Composition

Many of the natural pigments to be used in the present invention are water-soluble because they are in the form of dye, but in the present invention, it has been found that the natural pigment, and the dehydration condensation compound of a fatty acid and an amino acid are firmly complexed from the action form of coating, impregnation, and adsorption, and become insoluble in water as a water-insoluble pigment composition. The term "complexing" as used herein is not limited to the action form such as coating, impregnation, or adsorption, and is defined as not a simple mixture but a material that has been made insoluble in water by the interaction between the natural pigment and the dehydration condensation compound of a fatty acid and an amino acid by physical adsorption or chemical adsorption.

Water insolubilization obtained by the present invention can improve the resistance of the natural pigment to such a level that the natural pigment can be used as a coloring material equivalent to ordinary pigments in applications such as a food, a cosmetic, a coating material for a pharmaceutical or an agrochemical, or a printing marker, a stationery, a writing instrument, a printing ink, an inkjet ink, a metal ink, a paint, a plastic pigment a color toner, a fluorescent labeling agent, a fluorescent probe, or a chemical sensor. Furthermore, the insolubilization is expected to improve properties such as heat resistance and light resistance. Note that the use of the water-insoluble pigment composition of the present invention is not limited to the above use.

The water-insoluble pigment composition of the present invention can be used by setting a composition of a natural pigment and the dehydration condensation compound of a fatty acid and an amino acid, in terms of mass ratio, to natural pigment : dehydration condensation compound of fatty acid and amino acid = 0.1 : 99.9 to 90 : 10. Preferably, natural pigment : dehydration condensation compound of fatty acid and amino acid = 9 : 91 to 90 : 10 is satisfied.

The average particle size of the water-insoluble pigment composition of the present invention is preferably from 0.1 µm to 100 µm, and more preferably from 1 µm to 50 µm.

In the water-insoluble pigment composition of the present invention, the content of an element derived from the dehydration condensation compound of a fatty acid and an amino acid used is preferably 99% or less, more preferably 90% or less, and still more preferably 80% or less, relative to the dehydration condensation compound of a fatty acid and an amino acid itself.

### Method for Producing Water-Insoluble Pigment Composition

As a method for producing the water-insoluble pigment composition of the present invention, a method of mixing a natural pigment and a dehydration condensation compound of a fatty acid and an amino acid in a solvent is preferable because the most uniform water-insoluble pigment composition can be produced.

As a method of mixing the respective materials in a solvent for producing the water-insoluble pigment composition, 1) first, a dispersion or a solution of a dehydration condensation compound of a fatty acid and an amino acid is prepared. The solution is prepared by dissolving a dehydration condensation compound of a fatty acid and an amino acid using an aqueous sodium hydroxide solution or the like. The dispersion may be prepared by dispersing the dehydration condensation compound of a fatty acid and an amino acid in water, or by re-precipitating the dehydration condensation compound of a fatty acid and an amino acid by pH adjustment of the solution. 2) On the other hand, a natural pigment is dissolved in a solvent to prepare a solution. In the preparation of the dispersion or solution of 1 or 2, an alcohol may be used, or a mixed solvent of water and an alcohol may be used. 3) Next, the two solutions are mixed to prepare a water-insoluble pigment composition. The natural pigment solution may be mixed with the dispersion or solution of the dehydration condensation compound of a fatty acid and an amino acid, conversely, the dispersion or solution of the dehydration condensation compound of a fatty acid and an amino acid may be mixed with the natural pigment solution, or the two solutions may be mixed little by little to prepare the water-insoluble pigment composition. The mixing temperature may be room temperature, or the mixing may be performed by heating. In consideration of the decomposition temperature of the natural pigment alone, the mixing is preferably performed at 10 to 60°C, and more preferably 20 to 50°C. The pH of the dispersion or solution of the dehydration condensation compound of a fatty acid and an amino acid at the time of mixing is adjusted in consideration of the decomposition of the natural pigment. 4) The pH of the mixed liquid is adjusted to complex the natural pigment with the dehydration condensation compound of a fatty acid and an amino acid, thereby preparing a water-insoluble pigment composition.

The resulting mixed liquid is filtered and dried, whereby a water-insoluble pigment composition can be obtained. When the mixed liquid is filtered through a filter such as a Nutsche, a wet cake colored with the natural pigment is obtained on the filter paper, and thus it can be confirmed that the natural pigment and the dehydration condensation compound of a fatty acid and an amino acid are complexed. Furthermore, the wet cake of the water-insoluble pigment composition is repeatedly washed with water. The filtrate becomes colorless and transparent, and it can be similarly confirmed that the pigment component does not flow out. The obtained water-containing wet cake of the water-insoluble pigment composition is dried at room temperature, by heating, in a vacuum, by drying under reduced pressure, or the like, whereby a dry water-insoluble pigment composition can be obtained. The drying method and a dryer are not limited, and any ordinary method and apparatus may be used.

The water-insoluble pigment composition of the present invention can be used as a wet cake containing water or a dried water-insoluble pigment composition depending on the application. In a case where the water-insoluble pigment composition is used for an aqueous dispersion or ink, the wet cake can be used as it is, and in a case of a solvent dispersion, the wet cake can be used by replacing an aqueous system with a solvent system. The dried water-insoluble pigment composition may be used as it is, or may be used by being redispersed in water, an organic solvent, a resin solution, or the like.

### Stabilizer, Additive

It is of course possible to mix other organic pigments, inorganic pigments, dyes, or pigments in any proportions with the water-insoluble pigment composition of the present invention, and a desired hue can be satisfied. To further impart light resistance and heat resistance to the water-insoluble pigment composition of the present invention, a stabilizer or an additive can be added.

The stabilizer and the additive can be added to each or both of the dispersion of the dehydration condensation compound of a fatty acid and an amino acid and the natural pigment aqueous solution, or may be added to the prepared water-insoluble pigment composition.

The water-insoluble pigment composition of the present invention is mixed with another resin, rubber, additive, pigment, dye, or the like as necessary, and is adjusted and used for final foods, cosmetics, coating materials for pharmaceuticals or agrochemicals, or printing markers, stationery, writing instruments, printing inks, inkjet inks, metal inks, paints, plastic pigments, color toners, fluorescent labeling agents, fluorescent probes, chemical sensors, or the like. Hereinafter, an example of the above-described applications will be described.

### Cosmetic Application

The water-insoluble pigment composition of the present invention can be used as a cosmetic. The cosmetic for use is not particularly limited, and the water-insoluble pigment composition of the present invention can be used in various types of cosmetics.

The cosmetic may be any type of cosmetic as long as it can effectively exert the function. The cosmetic may be a lotion, a cream gel, a spray, or the like. Examples of the cosmetic include skin care cosmetics such as a face wash, a makeup remover, a skin lotion, a serum, a pack, a protective emulsion, a protective cream, a whitening cosmetic, and an ultraviolet protection cosmetic, makeup cosmetics such as a foundation, a face powder, a makeup base, a lipstick, an eye makeup, a blush, and a nail enamel, hair care cosmetics such as a shampoo, a hair rinse, a hair treatment, a hair styling agent, a permanent wave agent, a hair pigment, and a hair growth agent, and body care cosmetics such as a body washing cosmetic, a deodorant cosmetic, and a bath agent.

The amount of the water-insoluble pigment composition of the present invention to be used in the cosmetic can be appropriately set depending on the type of the cosmetic. The content in the cosmetic is usually in a range of 0.1 to 99 mass%, and generally, it is preferably in a range of 0.1 to 10 mass%. On the other hand, in a makeup cosmetic for coloring, the content is preferably in a range of 5 to 80 mass%, more preferably in a range of 10 to 70 mass%, and most preferably in a range of 20 to 60 mass%. When the amount of the water-insoluble pigment composition of the present invention contained in the cosmetic is within the above range, functions such as coloring properties can be effectively exhibited, and functions required for the cosmetic can also be maintained.

The cosmetic may include, in addition to the water-insoluble pigment composition of the present invention, a carrier, a pigment, an oil, a sterol, an amino acid, a moisturizer, a powder, a pH adjuster, a fragrance, an essential oil, a cosmetic active ingredient, a vitamin, an essential fatty acid, a sphingolipid, a self-tanning agent, an excipient, a filler, an emulsifier, an antioxidant, a surfactant, a chelating agent, a gelling agent, a thickener, an emollient, a humectant, a moisturizer, a mineral, a viscosity modifier, a flow modifier, a keratolytic agent, a retinoid, a hormone compound, an alpha-hydroxy acid, an alpha-keto acid, an anti-mycobacterial agent, an antifungal agent, an antibacterial agent, an antiviral agent, an analgesic, an antiallergic agent, an antihistamine, an anti-inflammatory agent, an anti-irritant, an antitumor agent, an immune system booster, an immune system inhibitor, an anti-acne agent, an anesthetic, a disinfectant, an insect repellent, a skin cooling compound, a skin protecting agent, a skin penetration enhancer, an exfoliant, a lubricant, an aromatic substance, a stain, a depigmentation agent, a hypopigmenting agent, a preservative, a stabilizer, a pharmaceutical, a light stabilizer, a spherical powder, and the like, which are acceptable as cosmetic components, depending on the type of cosmetic.

The cosmetic can be produced by mixing the water-insoluble pigment composition of the present invention and other cosmetic components. The cosmetic containing the water-insoluble pigment composition of the present invention can be used in the same manner as in ordinary cosmetics depending on the type of the cosmetic.

### Ink and Paint Applications

The water-insoluble pigment composition of the present invention can be used as an ink or a paint. However, the description will be made on the application and composition of the ink and the paint, but the present invention is not limited to these. The water-insoluble pigment composition of the present invention may be dispersed only in a thermoplastic resin, but may also be dispersed in a vehicle for a printing ink, a vehicle for a paint, or the like, which contains a thermoplastic resin as an essential component.

As the thermoplastic resin, for example, resins such as a polyester resin, a polyamide resin, a styrene resin, an acrylic resin, polyolefin, polyalkylene terephthalate, a polyvinyl chloride resin, and the like can be used as the resin for dispersion.

For example, a vehicle of a lithographic printing ink is produced from raw materials of 20 to 50% (by mass) of a resin such as a rosin-modified phenol resin, a petroleum resin, or an alkyd resin, 0 to 30% (by mass) of an animal or vegetable oil such as linseed oil, tung oil, or soybean oil, 10 to 60% (by mass) of a solvent such as n-paraffin, isoparaffin, naphthene, α-olefin, or aromatic, and several% (by mass) of an additional additive such as a solubilizing agent or a gelling agent.

In a case of a vehicle for a gravure printing ink or a flexographic printing ink, the vehicle is produced from raw materials including 10 to 50% (by mass) of one or more resins selected from a rosin, a maleic acid resin, a polyamide resin, a vinyl resin, a cyclized rubber, a chlorinated rubber, an ethylene-vinyl acetate copolymer resin, an urethane resin, a polyester resin, an alkyd resin, nitrocellulose, cellulose acetate, and the like, and 30 to 80% (by mass) of a solvent such as an alcohol, toluene, n-hexane, ethyl acetate, cellosolve, and butyl cellosolve acetate.

The vehicle for a paint is produced from raw materials including 20 to 80% (by mass) of a resin such as an alkyd resin, an epoxy resin, an acrylic resin, a polyurethane resin, a polyester resin, a melamine resin, a urea resin, or a water-soluble resin, and 10 to 60% (by mass) of a solvent such as a hydrocarbon, an alcohol, a ketone, or water.

### Plastic Application

The water-insoluble pigment compositions of the present invention can also be used in plastic coloring applications. In a case of obtaining a colored plastic molded article, a thermoplastic resin (plastic) for thermoforming such as injection molding or press molding, for example, a polyolefin such as polyethylene or polypropylene, or a polyvinyl chloride resin is used, and the water-insoluble pigment composition of the present invention can be used by being kneaded into such a resin by a method known in the related art.

### Toner Application

The water-insoluble pigment composition of the present invention can also be used for toner coloring applications. In a case of obtaining a toner for developing an electrostatic image, a film-forming thermoplastic resin which is solid at ordinary temperature, such as a polyester resin, a polyamide resin, a styrene resin, or an acrylic resin, is used as a resin for dispersion.

The toner for developing an electrostatic image produced using the water-insoluble pigment composition of the present invention as a constituent can be used as a one-component color magnetic toner containing a magnetic material in the toner (magnetic one-component developing color toner), a non-magnetic one-component color toner not containing a magnetic material (non-magnetic one-component developing color toner), or a color toner for a two-component color developer into which a carrier is mixed (two-component developing color toner).

The one-component color magnetic toner can be constituted by, for example, a pigment, a binder resin, a magnetic powder, and other additives typified by a charge control agent (CCA) and a release agent, as in the case of a commonly used toner.

The amount of the water-insoluble pigment composition used in the toner for developing an electrostatic image is not particularly limited, but is preferably 0.5 to 25 parts by mass relative to 100 parts by mass of the binder resin, and more preferably 4 to 10 parts by mass relative to 100 parts by mass of the binder resin from the viewpoint of making the charging performance of the pigment itself more remarkable.

As the binder resin to be used in the toner for developing an electrostatic image, any of the known and commonly used resins exemplified as the thermoplastic resin can be used, and any of synthetic resins, natural resins, natural rubbers, synthetic rubbers, synthetic waxes, and the like exhibiting adhesiveness under application of heat or pressure can be used.

### EXAMPLES

Hereinafter, the present invention will be described in more detail with reference to Examples, but the present invention is not limited to these Examples.

### Example 1

Into a 1-L beaker, 5.0 g of lauroyl lysine (N-lauroyl-L-lysine, available from FUJIFILM Wako Pure Chemical Corporation) and 50 g of ethanol were added and sufficiently blended. Next, 200 g of ion-exchanged water, 5.0 g of 48% aqueous sodium hydroxide solution (Cica Special Grade, available from Kanto Chemical Co., Inc.), and a stirring bar were added thereto, and the mixture was stirred with a magnetic stirrer for 30 minutes to obtain a lauroyl lysine solution. To a 3-L beaker, 1000 g of ion-exchanged water and 100 g of ethanol were added, and the mixture was stirred with a glass stirring blade connected to a three-one motor at room temperature for 5 minutes to prepare a mixed liquid of ion-exchanged water and ethanol. Into a 500-mL beaker, 15.0 g of annatto pigment (available from Kanto Chemical Co., Inc.), 300 g of ion-exchanged water, and a stirring bar were added and stirred using a magnetic stirrer at room temperature for 15 minutes to prepare an annatto pigment solution. The whole amount of the lauroyl lysine solution was added to the mixed liquid of ion-exchanged water and ethanol, and the whole amount of the annatto pigment solution was further added thereto. Subsequently, dilute hydrochloric acid prepared by diluting hydrochloric acid (Cica first grade, available from Kanto Chemical Co., Inc.) with ion-exchanged water by 10 times was slowly added dropwise with a dropper to adjust the pH to 4.0, followed by stirring at room temperature for 2 hours. This solution was filtered with a Nutsche, and the resulting solid was dried at 30°C for 14 hours in a vacuum dryer (740 mmHg) to obtain 11.5 g of a powder (1). The compositional ratio of lauroyl lysine and annatto pigment in the powder (1) was 1 : 3 in terms of the ratio of the amounts charged. The obtained powder exhibited an orange color of the same color system as that of the annatto pigment.

Into a 10-mL vial bottle, 100 mg of the powder (1), 1.0 g of ion-exchanged water, and a stirring bar were added and stirred with a magnetic stirrer for 5 minutes to prepare a dispersion (1). When one drop of the dispersion (1) was dropped on a filter paper, the dropped portion was colored orange in a circular shape, and then a state in which a colorless and transparent liquid was concentrically spread was observed. The portion colored orange was the powder (1) insolubilized in water, and the portion where the transparent liquid concentrically spread thereafter was water, and thus, it was confirmed that the powder (1) was insoluble in water.

### Example 2

Into a 1-L beaker, 20.0 g of lauroyl lysine (N-lauroyl-L-lysine, available from FUJIFILM Wako Pure Chemical Corporation) and 50 g of ethanol were added and sufficiently blended. Next, 200 g of ion-exchanged water, 10.0 g of 48% aqueous sodium hydroxide solution (Cica Special Grade, available from Kanto Chemical Co., Inc.), and a stirring bar were added thereto, and the mixture was stirred with a magnetic stirrer for 30 minutes to obtain a lauroyl lysine solution. To a 3-L beaker, 1000 g of ion-exchanged water and 100 g of ethanol were added, and the mixture was stirred with a glass stirring blade connected to a three-one motor at room temperature for 5 minutes to prepare a mixed liquid of ion-exchanged water and ethanol. To a 500-mL beaker, 2.0 g of sodium copper chlorophyllin (available from FUJIFILM Wako Pure Chemical Corporation), 300 g of ion-exchanged water, and a stirring bar were added and stirred using a magnetic stirrer at room temperature for 15 minutes to prepare a sodium copper chlorophyllin solution. Next, the whole amount of the lauroyl lysine solution was added to the mixed liquid of ion-exchanged water and ethanol, and dilute hydrochloric acid prepared by diluting hydrochloric acid (Cica first grade, available from Kanto Chemical Co., Inc.) with ion-exchanged water by 10 times was slowly added dropwise thereto with a dropper to adjust the pH to 7.0. The whole amount of the sodium copper chlorophyllin solution was added to the solution, and dilute hydrochloric acid was slowly added dropwise thereto with a dropper to adjust the pH to 4.0, followed by stirring at room temperature for 2 hours. This solution was filtered with a Nutsche, and the resulting solid was dried at 30°C for 14 hours in a vacuum dryer (740 mmHg) to obtain 19.4 g of a powder (2).

The compositional ratio of lauroyl lysine and sodium copper chlorophyllin in the powder (2) was 10 : 1 in terms of the ratio of the amounts charged. The obtained powder exhibited a green color of the same color system as that of sodium copper chlorophyllin.

Into a 10-mL vial bottle, 110 mg of the powder (2), 1.0 g of ion-exchanged water, and a stirring bar were added and stirred with a magnetic stirrer for 5 minutes to prepare a dispersion (2). When one drop of the dispersion (2) was dropped on a filter paper, the dropped portion was colored green in a circular shape, and then a state in which a colorless and transparent liquid was concentrically spread was observed. The portion colored green was the powder (2) insolubilized in water, and the portion where the transparent liquid concentrically spread thereafter was water, and thus, it was confirmed that the powder (2) was insoluble in water.

### Example 3

18.3 g of a powder (3) was obtained in the same manner as in Example 1, except that 2.0 g of red cabbage pigment (available from Kanto Chemical Co., Inc.) was used instead of the sodium copper chlorophyllin of Example 2. The compositional ratio of lauroyl lysine and red cabbage pigment in the powder (3) was 10 : 1 in terms of the ratio of the amounts charged. The obtained powder exhibited a red color of the same color system as that of the red cabbage pigment.

Into a 10-mL vial bottle, 110 mg of the powder (3), 1.0 g of ion-exchanged water, and a stirring bar were added and stirred with a magnetic stirrer for 5 minutes to prepare a dispersion (3). When one drop of the dispersion (3) was dropped on a filter paper, the dropped portion was colored red in a circular shape, and then a state in which a colorless and transparent liquid was concentrically spread was observed. The portion colored red was the powder (3) insolubilized in water, and the portion where the transparent liquid concentrically spread thereafter was water, and thus, it was confirmed that the powder (3) was insoluble in water.

### Example 4

The same experiment as in Example 1 was carried out except that LINABLUE G1 (available from DIC LIFETECH CORPORATION, trehalose 55%, spirulina extract 40%, trisodium citrate 5%) was used instead of the sodium copper chlorophyllin in Example 2, thereby obtaining 19.4 g of a powder (4). The compositional ratio of lauroyl lysine and spirulina extract in the powder (4) was 25 : 1 in terms of the ratio of the amounts charged. The obtained powder exhibited a blue color of the same color system as that of the phycocyanin pigment.

Into a 10-mL vial bottle, 104 mg of the powder (4), 1.0 g of ion-exchanged water, and a stirring bar were added and stirred with a magnetic stirrer for 5 minutes to prepare a dispersion (4). When one drop of the dispersion (4) was dropped on a filter paper, the dropped portion was colored blue in a circular shape, and then a state in which a colorless and transparent liquid was concentrically spread was observed. The portion colored blue was the powder (4) insolubilized in water, and the portion where the transparent liquid concentrically spread thereafter was water, and thus, it was confirmed that the powder (4) was insoluble in water.

### Example 5

Into a 1-L beaker, 11.0 g of N-capryloyllysine (n6-(1-oxooctyl)-L-lysine, available from Alfa Chemistry), and 50 g of ethanol were added and sufficiently blended. Next, 200 g of ion-exchanged water, 5.0 g of 48% aqueous sodium hydroxide solution (Cica Special Grade, available from Kanto Chemical Co., Inc.), and a stirring bar were added thereto, and the mixture was stirred with a magnetic stirrer for 30 minutes to obtain an N-capryloyl lysine solution. Into a 500-mL beaker, 3.3 g of annatto pigment (available from Kanto Chemical Co., Inc.), 300 g of ion-exchanged water, and a stirring bar were added and stirred using a magnetic stirrer at room temperature for 15 minutes to prepare an annatto pigment solution. Into a 3-L beaker, 1000 g of ion-exchanged water was added, the whole amount of the N-capryloyl lysine solution was added thereto, the whole amount of the annatto pigment solution was further added thereto. Subsequently, dilute hydrochloric acid prepared by diluting hydrochloric acid (Cica first grade, available from Kanto Chemical Co., Inc.) with ion-exchanged water by 10 times was slowly added dropwise with a dropper to adjust the pH to 4.0, followed by stirring at room temperature for 2 hours. This solution was filtered with a Nutsche, and the resulting solid was dried at 30°C for 14 hours in a vacuum dryer (740 mmHg) to obtain 11.5 g of a powder (5). The compositional ratio of N-capryloyl lysine and annatto pigment in the powder (5) was 110 : 33 in terms of the ratio of the amounts charged. The obtained powder exhibited an orange color of the same color system as that of the annatto pigment.

Into a 10-mL vial bottle, 143 mg of the powder (5), 1.0 g of ion-exchanged water, and a stirring bar were added and stirred with a magnetic stirrer for 5 minutes to prepare a dispersion (5). When one drop of the dispersion (5) was dropped on a filter paper, the dropped portion was colored orange in a circular shape, and then a state in which a colorless and transparent liquid was concentrically spread was observed. The portion colored orange was the powder (5) insolubilized in water, and the portion where the transparent liquid concentrically spread thereafter was water, and thus, it was confirmed that the powder (5) was insoluble in water.

### Comparative Example 1

Into a 10-mL vial bottle, 10 mg of annatto pigment (available from Kanto Chemical Co., Ltd.) and 1.0 g of water were added, and then a stirring bar was added thereto, followed by stirring for 5 minutes, thereby preparing a dispersion (6). When one drop of the dispersion (6) was dropped on a filter paper, it was observed that a yellow liquid was uniformly spread concentrically around the dropped portion. Here, it was confirmed that the annatto pigment was dissolved in water in the dispersion (6).

### Comparative Example 2

Into a 10-mL vial bottle, 75 mg of annatto pigment (available from Kanto Chemical Co., Inc.), 25 mg of lauroyl lysine (N-lauroyl-L-lysine, available from FUJIFILM Wako Pure Chemical Corporation), and 1.0 g of water were added, and then a stirring bar was added thereto, followed by stirring for 5 minutes, thereby preparing a dispersion (7). When one drop of the dispersion (7) was dropped on a filter paper, it was observed that a yellow liquid was uniformly spread concentrically around the dropped portion. Here, it was confirmed that the annatto pigment was dissolved in water in the dispersion (7).

### Comparative Example 3

Into a 10-mL vial bottle, 10 mg of sodium copper chlorophyllin (available from FUJIFILM Wako Pure Chemical Corporation) and 1.0 g of water were added, and then a stirring bar was added thereto, followed by stirring for 5 minutes, thereby preparing a dispersion (8). When one drop of the dispersion (8) was dropped on a filter paper, it was observed that a green liquid was uniformly spread concentrically around the dropped portion. Here, it was confirmed that sodium copper chlorophyllin was dissolved in water in the dispersion (8).

### Comparative Example 4

Into a 10-mL vial bottle, 10 mg of sodium copper chlorophyllin (available from FUJIFILM Wako Pure Chemical Corporation), 100 mg of lauroyl lysine (N-lauroyl-L-lysine, available from FUJIFILM Wako Pure Chemical Corporation), and 1.0 g of water were added, and then a stirring bar was added thereto, followed by stirring for 5 minutes, thereby preparing a dispersion (9). When one drop of the dispersion (9) was dropped on a filter paper, it was observed that a green liquid was uniformly spread concentrically around the dropped portion. Here, it was confirmed that sodium copper chlorophyllin was dissolved in water in the dispersion (9).

### Comparative Example 5

Into a 10-mL vial bottle, 10 mg of red cabbage pigment (available from Kanto Chemical Co., Ltd.) and 1.0 g of water were added, and then a stirring bar was added thereto, followed by stirring for 5 minutes, thereby preparing a dispersion (10). When one drop of the dispersion (10) was dropped on a filter paper, it was observed that a red liquid was uniformly spread concentrically around the dropped portion. Here, it was confirmed that the red cabbage pigment was dissolved in water in the dispersion (10).

### Comparative Example 6

Into a 10-mL vial bottle, 10 mg of red cabbage pigment (available from Kanto Chemical Co., Inc.), 100 mg of lauroyl lysine (N-lauroyl-L-lysine, available from FUJIFILM Wako Pure Chemical Corporation), and 1.0 g of water were added, and then a stirring bar was added thereto, followed by stirring for 5 minutes, thereby preparing a dispersion (11). When one drop of the dispersion (11) was dropped on a filter paper, it was observed that a red liquid was uniformly spread concentrically around the dropped portion. Here, it was confirmed that the red cabbage pigment was dissolved in water in the dispersion (11).

### Comparative Example 7

Into a 10-mL vial bottle, 10 mg of LINABLUE G1 (available from DIC LIFETECH CORPORATION, trehalose 55%, spirulina extract 40%, trisodium citrate 5%) and 1.0 g of water were added and then a stirring bar was added thereto, followed by stirring for 5 minutes, thereby preparing a dispersion (12). When one drop of the dispersion (12) was dropped on a filter paper, it was observed that a blue liquid was uniformly spread concentrically around the dropped portion. Here, it was confirmed that the phycocyanin pigment was dissolved in water in the dispersion (12).

### Comparative Example 8

Into a 10-mL vial bottle, 10 mg of LINABLUE G1 (available from DIC LIFETECH CORPORATION, trehalose 55%, spirulina extract 40%, trisodium citrate 5%), 100 mg of lauroyl lysine (N-lauroyl-L-lysine, available from FUJIFILM Wako Pure Chemical Corporation), and 1.0 g of water were added, and then a stirring bar was added thereto, followed by stirring for 5 minutes, thereby preparing a dispersion (13). When one drop of the dispersion (13) was dropped on a filter paper, it was observed that a blue liquid was uniformly spread concentrically around the dropped portion. Here, it was confirmed that the phycocyanin pigment was dissolved in water in the dispersion (13).

Into a 10-mL vial bottle, 33 mg of annatto pigment (available from Kanto Chemical Co., Inc.), 110 mg of N-capryloyl lysine (n6-(1-oxooctyl)-L-lysine, available from Alfa Chemistry), and 1.0 g of water were added, and then a stirring bar was added thereto, followed by stirring for 5 minutes, thereby preparing a dispersion (14). When one drop of the dispersion (14) was dropped on a filter paper, it was observed that a yellow liquid was uniformly spread concentrically around the dropped portion. Here, it was confirmed that the annatto pigment was dissolved in water in the dispersion (14).

The coloring materials of Examples and Comparative Examples were evaluated as a lipstick cosmetic, an eye cosmetic, a nail cosmetic, and a hair cosmetic.

### Evaluation Example, Comparative Evaluation Example

### Preparation and Evaluation of Lipstick Cosmetic

A balm cream base (available from Orangeflower K.K.) and castor oil (available from Orangeflower K.K.) were weighed into a pudding cup and mixed while heating in a hot water bath at 70°C to 80°C. The powder (1) prepared in Example 1, or the annatto pigment used in Comparative Example 1 was added thereto, and mixed while heating. The mixed liquid was filled in a silicon mold with a ring, allowed to stand for 10 minutes, and then cooled in a refrigerator at 10°C for about 10 minutes. The lipstick cosmetic protruding from the ring was shaved with a spatula, and after removing the ring, a lipstick cylinder was inserted. A lower portion of the silicone mold was pinched, the cylinder was gradually lowered by introducing a small amount of air, and the silicone mold was housed in the cylinder to obtain a lipstick cosmetic. The lipstick cosmetics thus prepared are summarized in Table 1.

**[Table 1]**

| Evaluation Example/Comparative Evaluation Example | Color material | Amount added (g) | | | |
|---|---|---|---|---|---|
| | | Color material | Cream base | Castor oil | Total |
| Evaluation Example 1 | Powder (1) | 0.35 | 6.00 | 0.35 | 6.70 |
| Comparative Evaluation Example 1 | Annatto pigment | 0.35 | 6.00 | 0.35 | 6.70 |
| Comparative Evaluation Example 2 | Annatto pigment | 0.032 | 6.32 | 0.35 | 6.70 |

The water resistance, coarse grains in appearance, feeling of use, color unevenness, and cosmetic durability of each lipstick cosmetic were evaluated. The water resistance was evaluated by applying the lipstick cosmetic to a filter paper, 1 mL of water was dropped on the colored portion with a dropper, and the degree of bleeding was visually checked. The coarse grains in appearance was evaluated by visually checking the lipstick cosmetic. The feeling of use and the color unevenness were evaluated by applying the lipstick cosmetic to the wrist. The cosmetic durability was evaluated by visually checking the color loss when the lipstick cosmetic applied to the wrist was rubbed three times with tissue paper. The evaluation results are summarized in Table 2. The water resistance was evaluated as "o" when no bleeding occurred, and "×" when bleeding occurred. The coarse grains in appearance was evaluated as "∘" when there was no coarse grain, and "×" when there were coarse grains. The feeling of use was evaluated as "o" when it was good, and as "×" when it was poor. The color unevenness was evaluated as "o" when there was no unevenness in coloring, and evaluated as "×" when there was unevenness in coloring. The cosmetic durability was evaluated as "o" when there was no color loss, and "×" when there was color loss.

**[Table 2]**

| Evaluation Example/Comparative Evaluation Example | Color material | Evaluation item | | | | |
|---|---|---|---|---|---|---|
| | | Water resistance | Coarse grains in appearance | Feeling of use | Color unevenness | Cosmetic durability |
| Evaluation Example 1 | Powder (1) | ○ | ○ | ○ | ○ | ○ |
| Comparative Evaluation Example 1 | Annatto pigment | × | × | × | × | × |
| Comparative Evaluation Example 2 | Annatto pigment | × | × | × | × | × |

As is clear from Table 2, the lipstick cosmetics using the water-insoluble pigment composition of the present invention showed superiority in terms of water resistance, coarse grains in appearance, feeling of use, color unevenness, and cosmetic durability, as compared with the lipstick cosmetics using the natural pigment as it was.

### Preparation and Evaluation of Eye Cosmetic

14.1 g of talc (Yamaguchi Mica Co., Ltd.), 0.02 of methylparaben (Maruzen Chemicals Co., Ltd.), 0.02 g of propylparaben (Maruzen Chemicals Co., Ltd.), 1.88 g of trihydroxystearin (Matsumoto Trading Co., Ltd.) were weighed and placed in a coffee grinder, and stirred for 10 seconds three times to prepare a dispersion base. The dispersion base and the powder (1) prepared in Example 1 or the annatto pigment used in Comparative Example 1 were added to a coffee grinder, and stirred for 5 seconds twice to prepare an eye cosmetic powder. The eye cosmetic powder was placed on a metal dish and pressed to prepare an eye cosmetic. The eye cosmetics thus prepared are summarized in Table 3.

**[Table 3]**

| Evaluation Example/Comparative Evaluation Example | Color material | Amount added (g) | | |
|---|---|---|---|---|
| | | Color material | Dispersion base | Total |
| Evaluation Example 2 | Powder (1) | 0.75 | 4.25 | 5 |
| Comparative Evaluation Example 3 | Annatto pigment | 0.75 | 4.25 | 5 |
| Comparative Evaluation Example 4 | Annatto pigment | 0.068 | 4.93 | 5 |

The water resistance, roughness, color unevenness, and cosmetic durability of each of the eye cosmetics were evaluated. The water resistance was evaluated by applying the eye cosmetic to a filter paper, dropping 0.5 mL of water on the colored portion with a dropper, and visually observing the degree of bleeding. The roughness and the color unevenness were evaluated by applying the eye cosmetic to the wrist. The cosmetic durability was evaluated by the coloration after rubbing the eye cosmetic applied to the wrist three times. The evaluation results are summarized in Table 4. The water resistance was evaluated as "o" when no bleeding occurred, and "×" when bleeding occurred. The roughness was evaluated as "o" when there was no roughness, and "×" when there was roughness. The color unevenness was evaluated as "o" when there was no unevenness in coloring, and evaluated as "×" when there was unevenness in coloring. The cosmetic durability was evaluated as "o" when there was no color loss, and "×" when there was color loss.

**[Table 4]**

| Evaluation Example/Comparative Evaluation Example | Color material | Evaluation item | | | |
|---|---|---|---|---|---|
| | | Water resistance | Roughne ss | Color unevenness | Cosmetic durability |
| Evaluation Example 2 | Powder (1) | ○ | ○ | ○ | ○ |
| Comparative Evaluation Example 3 | Annatto pigment | × | × | × | × |
| Comparative Evaluation Example 4 | Annatto pigment | × | × | × | × |

As is clear from Table 4, the eye cosmetic using the water-insoluble pigment composition of the present invention showed superiority in terms of water resistance, roughness, color unevenness, and cosmetic durability, as compared with the eye cosmetic using the natural pigment as it was.

### Preparation and Evaluation of Nail Cosmetic

Into a 20-mL plastic bottle, 1.7 g of the powder (1) prepared in Example 1, or the annatto pigment used in Comparative Example 1 and a nail holic base coat (color: SP030, available from KOSE Corporation) were added and mixed with a dropper. A nail holic base coat was added thereto to prepare a nail cosmetic. The nail cosmetics thus prepared are summarized in Table 5.

**[Table 5]**

| Evaluation Example/Comparative Evaluation Example | Color material | Amount added (g) | | |
|---|---|---|---|---|
| | | Color material | Nail holic base coat | Total |
| Evaluation Example 3 | Powder (1) | 0.4 | 4.6 | 5 |
| Comparative Evaluation Example 5 | Annatto pigment | 0.4 | 4.6 | 5 |
| Comparative Evaluation Example 6 | Annatto pigment | 0.09 | 4.91 | 5 |

The water resistance, the unevenness of the coated surface, and the color unevenness of each nail cosmetic were evaluated. The water resistance was evaluated by applying the nail cosmetic to a filter paper, dropping 1 mL of water on the colored portion of the filter paper using a dropper, and visually observing the degree of bleeding. The color unevenness and the unevenness of the coated surface were evaluated by applying the nail cosmetic to a nail chip, drying the nail cosmetic, and then checking the appearance and the texture of the coated surface. The evaluation results are summarized in Table 6. The water resistance was evaluated as "o" when no bleeding occurred, and "×" when bleeding occurred. The unevenness of the coated surface was evaluated as "o" when there was no unevenness, and as "×" when there was unevenness. The color unevenness was evaluated as "o" when there was no unevenness in coloring, and evaluated as "×" when there was unevenness in coloring.

**[Table 6]**

| Evaluation Example/Comparative Evaluation Example | Color material | Evaluation item | | |
|---|---|---|---|---|
| | | Water resistance | Unevenness of coated surface | Color unevenness |
| Evaluation Example 3 | Powder (1) | ○ | ○ | ○ |
| Comparative Evaluation Example 5 | Annatto pigment | × | × | × |
| Comparative Evaluation Example 6 | Annatto pigment | × | × | × |

As is clear from Table 6, the nail cosmetics using the water-insoluble pigment composition of the present invention showed superiority in terms of water resistance, unevenness of the coated surface, and color unevenness, as compared with the nail cosmetics using the natural pigment as it was.

### Preparation and Evaluation of Hair Color Cream

A hair color cream was prepared and evaluated as a hair cosmetic. In a 50-mL beaker, distilled water, propylene glycol (available from KOYO Fine Chemical Corporation), polyquaternium-37·dicaprylyl carbonate-lauryl glucoside (available from BASF Japan Ltd.), and 0.016 g of EDTA-2Na (available from BASF Japan Ltd.) were weighed, and mixed with a stirrer until they became uniform. The powder (1) prepared in Example 1, or the annatto pigment used in Comparative Example 1 was added thereto and mixed with a spatula until the mixture became uniform. Subsequently, methylparaben (available from Maruzen Chemicals Co., Ltd.) and propylparaben (available from Maruzen Chemicals Co., Ltd.) were added and mixed with a spatula until the mixture became uniform, thereby preparing a hair color cream. The hair color creams thus prepared are summarized in Table 7.

**[Table 7]**

| Evaluation Example/Comparative Evaluation Example | Color material | Amount added (g) | | | | | |
|---|---|---|---|---|---|---|---|
| | | Color material | Distilled water | Propylene glycol | Polyquaternium-37·dicaprylyl carbonate·lauryl glucoside | EDTA-2Na | Total |
| Evaluation Example 4 | Powder (1) | 3.2 | 11.4 | 1.1 | 0.28 | 0.02 | 16 |
| Comparative Evaluation Example 7 | Annatto pigment | 3.2 | 11.4 | 1.1 | 0.28 | 0.02 | 16 |
| Comparative Evaluation Example 8 | Annatto pigment | 0.29 | 13.9 | 1.4 | 0.34 | 0.02 | 16 |

### Hair Color Evaluation Method

### Color Development Evaluation

### Color development when applied to blond human hair

The hair color cream was spread on blond human hair by hand and the color development was evaluated visually. The color development on the blond hair was evaluated as "o" when the color development was good, and as "×" when the color development was poor.

### Color development when applied to black human hair

The hair color cream was spread on black human hair by hand, and the color development was evaluated visually. The color development on black hair was evaluated as "o" when the color development was good, and as "×" when the color development was poor.

### Color development when applied to artificial skin

The hair color cream was applied and spread on the artificial skin with a finger in such a manner to draw a circle, and the color development was evaluated visually. The color development on the artificial skin was evaluated as "o" when the color development was good, and as "×" when the color development was poor.

### Evaluation of Water Resistance

The hair color cream was applied and spread on a filter paper with a finger in such a manner to draw a circle, and dried at room temperature for about 10 minutes. Then, 1 mL of tap water was dropped on the colored central portion with a dropper. At this time, in a case where the dropped portion was colored without changing from the state before dropping and a state in which a colorless and transparent solution was concentrically spread from the colored portion was observed, it was evaluated that the hair color cream did not bleed into water. The water resistance was evaluated as "o" when no bleeding occurred, and "×" when bleeding occurred.

### Evaluation of Pigment Deposition

The hair color cream was applied evenly to blond human hair and dried at room temperature for about 10 minutes. The blond human hair was washed by hand with a half push of a shampoo (LUX super rich shine damage repair wet repair shampoo available from Unilever Japan K.K.), and the shampoo was thoroughly washed away with tap water at room temperature. Further, a conditioner (LUX super rich shine damage repair wet repair conditioner/Unilever Japan K.K.) was applied by a half push, and then washed away with tap water at room temperature. After washing, the blond human hair was dried with hot air from a dryer for about 2 minutes, and then the presence or absence of color remaining was confirmed. The presence or absence of pigment deposition was confirmed. A case where pigment deposition was not confirmed was evaluated as "o", and a case where pigment deposition was confirmed was evaluated as "×". The evaluation results of the hair cosmetics prepared are summarized in Table 8.

**[Table 8]**

| Evaluation Example/ Comparative Evaluation Example | Color material | Evaluation item | | | | |
|---|---|---|---|---|---|---|
| | | Color development on blond hair | Color development on black hair | Color development on artificial skin | Water resistance | Pigment deposition |
| Evaluation Example 4 | Powder (1) | ○ | ○ | ○ | ○ | ○ |
| Comparative Evaluation Example 7 | Annatto pigment | ○ | × | × | × | × |
| Comparative Evaluation Example 8 | Annatto pigment | ○ | × | × | × | × |

As is clear from Table 8, the hair cosmetics using the water-insoluble pigment composition of the present invention showed superiority in terms of color development on black hair, color development on artificial skin, water resistance, and pigment deposition, as compared with the hair cosmetics using the natural pigment as it was.

As described above, the lipstick cosmetic, the eye cosmetic, the nail cosmetic, and the hair cosmetic, in which the water-insoluble pigment composition of the present invention was blended, exhibited superior performance as compared with the cosmetic in which the natural pigment was blended as it was. It is considered that the water-insoluble pigment composition of the present invention exhibited superior performance by complexing the natural pigment, and the dehydration condensation compound of a fatty acid and an amino acid, and the powders described in Examples exhibited superior performance as cosmetics.

## Claims

1. A water-insoluble pigment composition obtained by complexing a natural pigment, and a dehydration condensation compound of a fatty acid and an amino acid.

2. The water-insoluble pigment composition according to claim 1, wherein a mass ratio of the natural pigment and the dehydration condensation compound of the fatty acid and the amino acid satisfies natural pigment : dehydration condensation compound of fatty acid and amino acid = 0.1 : 99.9 to 90 : 10.

3. The water-insoluble pigment composition according to claim 1 or 2, wherein the dehydration condensation compound of the fatty acid and the amino acid is one or more selected from lauroyl lysine and N-capryloyl lysine.

4. The water-insoluble pigment composition according to claim 1 or 2, wherein the natural pigment is at least one or more selected from a carotenoid-based pigment, a porphyrin-based pigment, a flavonoid-based pigment, and a chromoprotein.

5. The water-insoluble pigment composition according to claim 1 or 2, wherein the natural pigment is at least one or more selected from an annatto pigment, a chlorophyll pigment, a safflower yellow pigment, and a phycocyanin pigment.

6. A food, a cosmetic, a lipstick cosmetic, an eye cosmetic, a nail cosmetic, a base makeup cosmetic, a hair cosmetic, a pharmaceutical, a coating material for agricultural chemicals, a printing marker, a stationery, a writing instrument, a printing ink, an inkjet ink, a metal ink, a paint, a plastic pigment, a color toner, a fluorescent labeling agent, a fluorescent probe, or a chemical sensor, comprising the water-insoluble pigment composition according to claim 1 or 2.
